# EUROPEAN PATENT APPLICATION

(11) **EP 0 551 200 A1**
(43) Date of publication of application: **14.07.1993**
(21) Application number: 93300105.9
(22) Date of filing: 07.01.1993
(51) Int. Cl.: A61K 31/35, A61K 35/00, C12N 9/99

(54) **Protein phosphatase inhibitors for use in therapy**

(30) Priority: 07.01.1992 GB 9200248; 22.01.1992 GB 9201390
(71) Applicant: NATIONAL UNIVERSITY OF SINGAPORE, Singapore 0511 (SG)
(72) Inventor: Cao, Xinmin, Singapore 0511 (SG); Guy, Graeme, Singapore 0511 (SG); Tan, Yin Hwee, Singapore 0511 (SG)
(74) Representative: Woods, Geoffrey Corlett

(57) **Abstract**

Protein phosphatase inhibitors such as okadaic acid, a phosphatase inhibitor dervied from a marine black sponge, and calyculin A are able to mimic tumor necrosis factor or interleukin-1. These phosphatase inhibitors may therefore be used to treat conditions which are treatable by tumor necrosis factor or interleukin-1 such as an advanced cancer or a leukaemia.

## Description

The present invention relates to uses of protein phosphatase inhibitors, for example inhibitors of protein phosphatases 1 and 2A such as okadaic acid and calyculin A.

The control of the immune system is mediated through a complex interplay of lymphokines with different kinds of cells. Tumour necrosis factor (TNF) and interleukin-1 (IL-1) are examples of lymphokines reported to be involved in the regulation of the host immune system. TNF and IL-1 are associated with disease states such as sepsis and autoimmune disorders and are described as mediators of the inflammatory response. Elevated levels of serum TNF in cancer, AIDS and malaria are related to weight loss in these diseases. The association of these lymphokines with the disease states has prompted the application of TNF antibodies or inhibitors in the treatment of septic shock and the use of IL-1 antibodies in rheumatoid arthritis.

IL-1 was originally described as a comitogen of thymocytes and TNF was first reported to be an endotoxin-induced factor which causes tumor necrosis. The two lymphokines have similar biological activities on cells even though they do not have homologous amino acid sequences or homology in their receptors. Unlike the epidermal growth factor (EGF), platelet derived growth factor (PDGF) and colony-stimulating factor-1 receptors which are known to have tyrosine kinase activity and "serpentine receptors" that generate second messengers via GTP-binding proteins, little has been deduced of the mode of TNF or IL-1 signal transduction from their receptors. The involvement of GTP-binding proteins in TNF and Il-1 signalling processes was implied from several reports. Increases in second messenger concentrations of cAMP, inositol 1,4,5-trisphosphate, and arachidonic acid have also been reported. The identification of several phosphorylated cellular substrates like the heat shock proteins, 1-plastin, stathmin, talin and EGF receptor (EGFR) in TNF or IL-1 stimulated cells suggested the activation of serine/threonine kinase(s). In this connection, both lymphokines have been reported to concordantly induce early changes in the phosphorylation of 63 cytosolic proteins and to activate multiple protein kinases including microtubule-associated protein-2 and eIF-4E kinases in human fibroblasts.

TNF or IL-1 activates a number of transcription factors including NF-kB, NF-IL6, multiple response factor, interferon response factor-1, interferon response factor-2, c-fos, c-jun, and NF-GMa and induces the expression of several genes. Much has been described of TNF and IL-1 signalling effects at the transmembrane, cytosol and gene expression levels, but the primary signalling pathway remains unresolved and in certain cases contradictory.

The immediate early gene Egr-1 (also known as NGF1-A, zif268, TIS8 and Krox24) is rapidly and transiently induced during the transition of cells from the G₀ to G₁ phase in response to serum or purified growth factor stimulation. It encodes a nuclear phosphoprotein with a DNA binding domain and functions as a transcriptional factor. Egr-1 expression is regulated by zinc-finger containing proteins (products of the Wilms tumor gene and the Egr-1 gene itself) which bind to the sequence 5'CGCCCCCGC-3' located in the Egr-1 promoter region. The Wilms tumor gene product, a putative tumor suppressor, represses Egr-1 expression whereas the Egr-1 protein induces its own expression in NIH 3T3 cells. Human T-cell leukemia virus type 1 (HTLV-1) is linked to adult T-cell leukemia and Egr-1 is expressed at a high level in several HTLV-1 infected T-cell lines in which the transregulatory protein (Tax) of HTLV-1 is constitutively expressed. Furthermore, Egr-1 expression has been shown to be transactivated by Tax. These findings suggest that Egr-1 gene expression is associated with cell growth and with cell transformation. Activation of protein kinase C is reported to play a part in the induction of Egr-1 gene expression.

US-A-4835176 discloses glycookadaic acid for use in treating cachexia-like side effects caused by TNF in a mammalian animal.

We have now found that okadaic acid, a phosphatase inhibitor derived from a marine black sponge (Tachibana et al, J. Am. Chem. Soc. 103, 2469-2471, 1981), induces a composite pattern of early phosphorylation changes and gene expression in primary human fibroblasts strikingly in common with those induced by TNF or IL-1. We have also found that okadaic acid and another phosphatase inhibitor, calyculin A, strongly induce a sustained expression of the Egr-1 gene in human and mouse fibroblasts and that okadaic acid and phorbol 12-myristate 13-acetate, a protein kinase activator, could synergistically mediate the induction of the Egr-1 gene. These findings have general applicability.

Accordingly, the present invention provides the use of a protein phosphatase inhibitor in the preparation of a medicament for use as a mimic of TNF or IL-1. The invention also provides the use of a protein phosphatase inhibitor in the preparation of a medicament for use as a cell differentiation factor. The invention further provides products containing a protein phosphatase inhibitor and a protein kinase activator as a combined preparation for simultaneous, separate or sequential use in inducing cell differentiation.

The protein phosphatase inhibitor is typically a protein serine/threonine phosphatase inhibitor such as an inhibitor of protein phosphatase 1 or 2A. The inhibitor may be okadaic acid or a pharmaceutically acceptable salt, amide or ester thereof. The salt may be an alkali metal or alkaline earth metal salt such as the sodium or potassium salt. The amide may be the simple acid amide, -CONH₂, or a mono- or di-alkyl amide such as an amide denoted by the formula -CONR₁R₂ wherein R₁ is hydrogen or C₁-C₄ alkyl and R₂ is C₁-C₄ alkyl. Alternatively, the amide may be an amide with an amino acid, for example an amide between okadaic acid and glycine or lysine. The amide with glycine is glycookadaic acid. The amide between okadaic acid and an amino acid may be in the form of a pharmaceutically acceptable salt. The ester may be a C₁-C₄ alkyl ester such as the methyl or ethyl ester of okadaic acid.

An alternative inhibitor for use in the invention is calyculin A or a derivative thereof. Any functional derivative capable of mimicking TNF or IL-1 or of inducing induction of the Egr-1 gene may be used. The derivative may be the acid or amines of calyculin A.

The following protein phosphatase inhibitors may therefore be used in the present invention: okadaic acid, calyculin A, dinophysistoxin-1, okadaic acid tetramethyl ether, acanthifolicin, 7-0-palmitoylokadaic acid, 7-0-docosahexaenoylokadaic acid, glycookadaic acid, okadylamine, okadanol, nor-okadanol, okadaic acid glycol, and okadaic acid spiroketal II.

As a mimic of TNF or IL-1, the protein phosphatase inhibitor may be employed in the treatment of a disease state treatable by TNF or IL-1. The inhibitor may therefore be used in the treatment of a tumour. The tumour can be selected from an advanced cancer such as a metastatic cancer, melanoma, renal cell carcinoma, leukaemia, broncogenic carcinoma, nasopharyngeal carcinoma and Kaposi sarcoma. The advanced cancer may be a metastatic carcinoma or sarcoma. The leukaemia may be adult leukaemia. Preferably the tumour is treated after debulking of the main tumour mass. The main tumour mass may be removed by surgery, radiotherapy and/or chemotherapy.

The protein phosphatase inhibitor may also be employed as a cell differentiation factor. The inhibitor can be used to promote cell growth. The inhibitor may act by induction of the Egr-1 gene. The inhibitor may therefore be used to treat a leukaemia, for example adult leukaemia. The induction of differentiation makes leukaemia less aggressive (Sachs, Nature 274, 535-539, 1978; Degas, Leukemia Research 14, 717-719, 1990). Preferably the tumour is treated after debulking of the main tumour mass as above.

The protein phosphatase inhibitor may be used in inducing cell differentiation and growth in conjunction with a protein kinase activator, for example an activator of protein kinase C. The inhibitor and the activator may be used to promote cell growth. The protein kinase activator may be administered at the same time as the protein phosphatase inhibitor, or at an earlier or later time.

A suitable activator of protein kinase C is phorbol 12-myristate 13-acetate whose alternative name is O-tetradecanoylphorbol 13-acetate (TPA). 1,2-Diglycerides of short chain (C-6, C-8 and C-10) fatty acids are also activators of protein kinase C, for example 1,2-dihexanoyl-sn-glycerol (C6:0) and 1,2-dioctanoyl-sn-glycerol (C8:0; alternative name 1,2-dicapryloyl-sn-glycerol). Other activators of protein kinase C are 1-oleoyl-2-acetyl-rac-glycerol (C18:1, [cis]-9/C2:0; alternative name 1-[(cis)-9-octadecenoyl]-2-acetyl-rac-glycerol), 1-oleoyl-2-acetyl-sn-glycerol and 1-stearoyl-2-arachidonoyl-sn-glycerol (C18:0/C20:4, [cis, cis, cis, cis]-5,8,11,14; alternative name 1-octadecanoyl-2-[(cis, cis, cis, cis)-5,8,11,14-eicosa-tetraenoyl]-sn-glycerol).

An agent for use as a mimic of TNF or IL-1 or for use as a cell differentiation and growth promoter can therefore be provided. The agent comprises a protein phosphatase inhibitor. A pharmaceutical composition may be formulated, comprising the inhibitor and a pharmaceutically acceptable carrier or diluent. The protein phosphatase inhibitor may be given by oral or rectal administration or by injection.

When an injectable preparation is produced, a pH controlling agent, a buffer, a stabilizing agent and/or an excipient may be added. Further, according to conventional techniques, lyophilized injectable preparations may be produced. There can be produced preparations for subcutaneous, intramuscular or intravenous injection.

Orally administrable solid preparations may be produced by adding an excipient and, if desired, a binding agent, disintegrator, lubricating agent, coloring agent, and/or flavoring agent, and the like to the inhibitor, and forming tablets, coated tablets, granules, powders or capsules according to conventional technique. Oral liquid compositions may be prepared by adding a flavoring agent, buffer, and/or stabilizer to the main drug and forming a syrup or dry syrup.

A rectal suppository may be prepared by adding an excipient and, if desired, a surfactant to the inhibitor to form the rectal suppository according to conventional technique.

The protein kinase activator, if employed, may be formulated with the protein phosphatase inhibitor as above. Alternatively, the activator may be formulated separately with a pharmaceutically acceptable carrier or diluent.

In use, a therapeutically effective amount of the protein phosphatase inhibitor is administered to a host. The host is typically a human but may be another mammal. The condition of a patient may thus be improved. A disease from which a patient suffers may be ameliorated. The dosage levels for the protein phosphatase inhibitor differ depending on a variety of factors including condition of the patient, the age and body weight of the patient, the disease state being treated and the administration form. Dosage levels of the order of about 0.05-500 mg per day are useful for injection though the dosage levels may differ from portion to portion. When administered orally or rectally, the dosage levels are about 0.05-1000 mg per day. The dosage levels may be changed according to the requirements of a doctor.

A therapeutically effective amount of a protein-kinase activator may be administered to a host in conjunction with the inhibitor. The activator may be given up to several days before, simultaneously with or up to several days after the inhibitor. The inhibitor may therefore be given up to seven days, for example up to two days, either side of the administration of the inhibitor.

The route by which the activator is given and the dosage depends on a variety of factors, including the particular activator being administered. Dosage may depend too on the age, weight and condition of the patient. Generally speaking, though, the activator may be administered by injection, for example intravenously, at a dosage level of about 0.05-500 mg per day. When administered orally or rectally, a dosage level of from 0.05 to 1000 mg per day may be suitable.

As a mimic of TNF, a protein phosphatase inhibitor can also be used to create an animal model for arthritis. Such a model enables treatment of this disease to be further studied. The invention therefore also provides a method of inducing arthritis in an animal, which method comprises administering thereto an amount of a protein phosphatase inhibitor sufficient to induce arthritis therein.

Any protein phosphatase inhibitor as described above may be employed. The animal may be a rodent, such as a mouse or rat, or a pig. Any laboratory animal which may develop arthritis can be employed. The protein phosphatase inhibitor is typically administered by injection, for example intravenously or intraperitoneally. A dose of from 0.0001 to 0.1 µg of the inhibitor per kg body-weight of the animal may be given by injection, for example intravenously or intraperitoneally, daily for from 5 to 10 days.

The following Examples illustrate the invention. In the accompanying drawings:

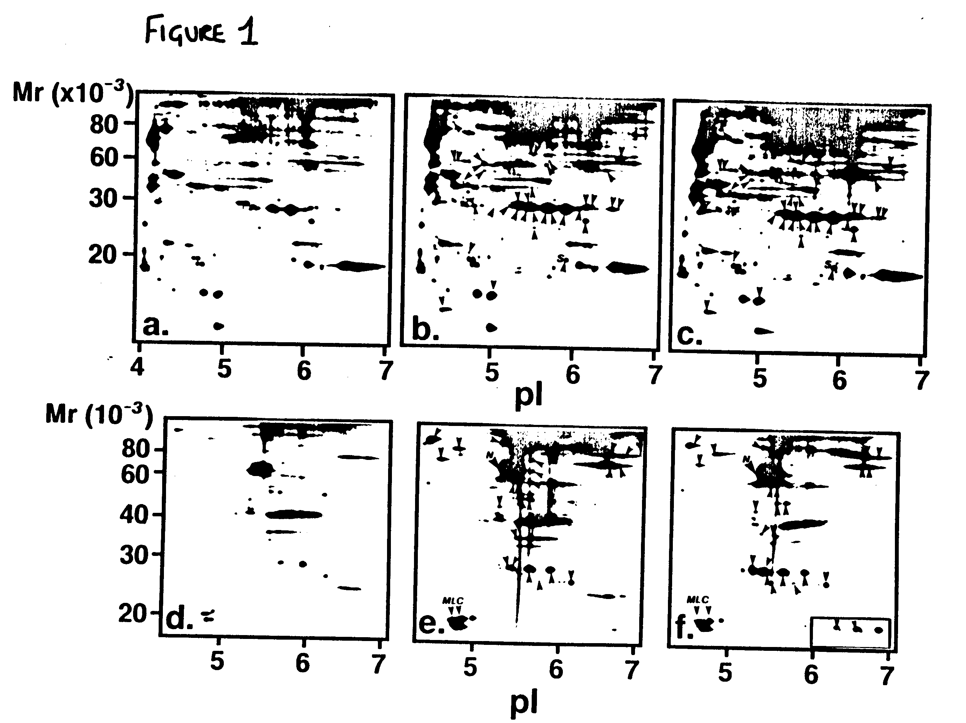

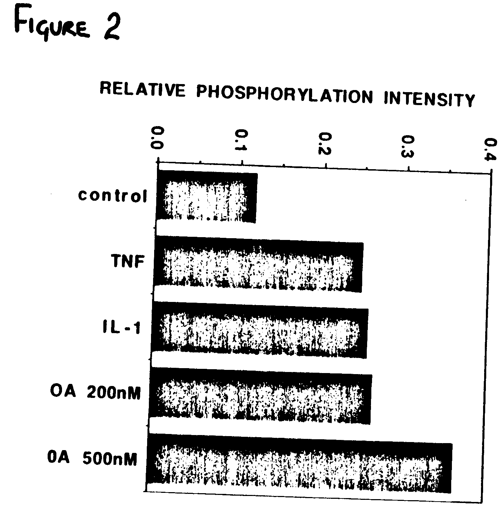

Fig. 1 shows a comparison of agonist-induced changes in early protein phosphorylation in human fibroblasts. ³²P-Labeled cultures were treated with TNF or okadaic acid (OA) for 15 min. The cells were then subjected to analysis by high resolution two-dimensional gel electrophoresis combined with computerized imaging as previously described (Guy et al, J. Biol. Chem. 266, 14343-14352, 1991). Autoradiographs from control cytosol (a), TNF (50 units/ml)-treated cytosol (b), 200 nM OA-treated cytosol (c), untreated nuclear/cytoskeletal extract (d), TNF (50 units/ml) treated nuclear/cytoskeletal extract (e), and 200 nM OA-treated nuclear/cytoskeletal extract (f). Arrows indicate the prominent increases in phosphorylation over the control. A few spots are "lost" during photography. The insert in (f) highlights increases in phosphorylation of the hsp complex in nuclear/cytoskeletal extracts derived from cells treated with a lower concentration of OA (100 nM).

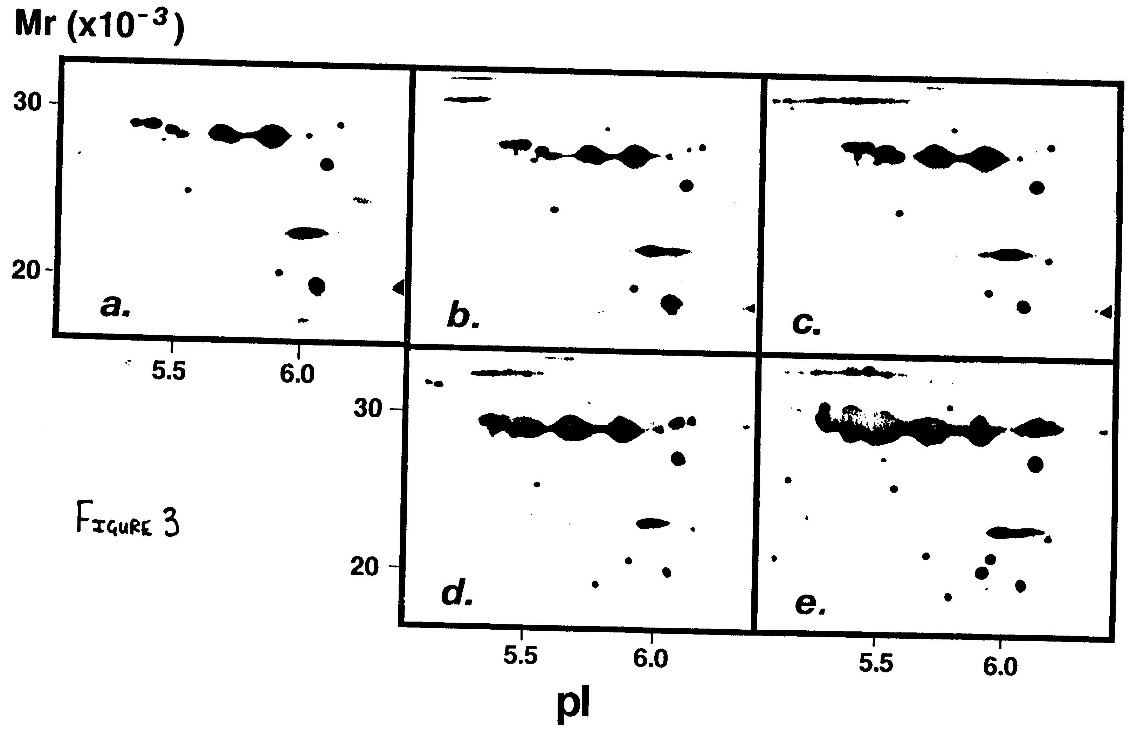

Fig. 2 shows the phosphorylation of cap-binding protein by cytosolic extracts of human fibroblasts treated with TNF (50 units/ml), IL-1 (50 units/ml), or OA (okadaic acid) at the indicated concentration for 15 min. The ³²P-labeled cap-binding protein (27,000 kDa) as purified by SDS-PAGE was quantified by computerized densitometry and compared in relative phosphorylation intensity.

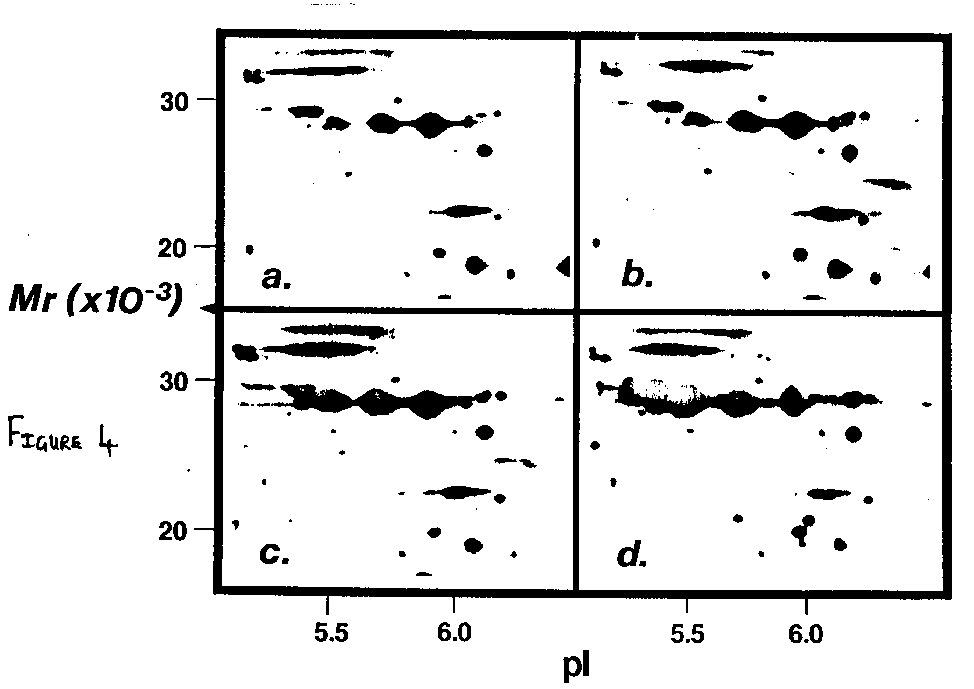

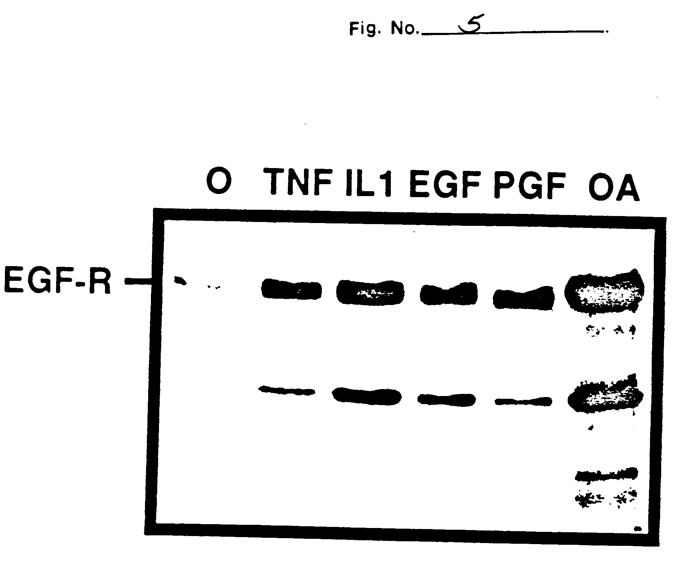

Fig. 3 shows the dose-dependent phosphorylation of the 27 hsp complex by okadaic acid or TNF. Human fibroblasts prelabeled with [³²P]orthophosphate (1 mCi/ml) were incubated with increasing concentrations of OA or TNF. Cytosolic fractions were prepared from the cells and subjected to two-dimensional gel electrophoresis. a, control; b, 80 nM OA; c, 160 nM OA; d, 50 units/ml TNF; and e, 320 nM OA.

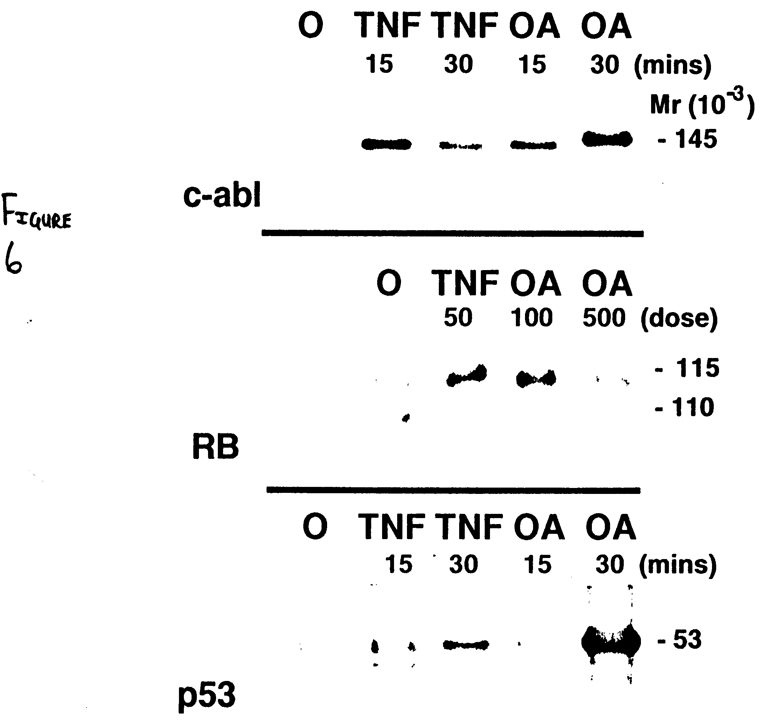

Fig. 4 shows the time-course of phosphorylation of the 27 hsp complex of the cytosolic fraction of human fibroblasts prelabeled with [³²P]orthophosphate (1 mCi/ml) by okadaic acid. The fibroblasts were treated with 200 nM of okadaic acid for 0 min (a), 5 min (b), 15 min (c), or 30 min (d). Cytosolic fractions were prepared from the fibroblasts and analyzed by two-dimensional gel electrophoresis.

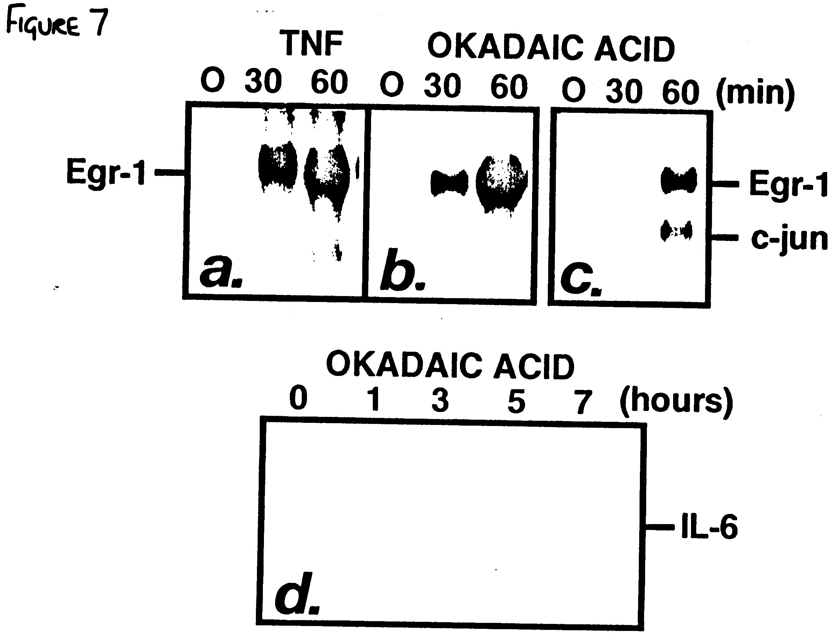

Fig. 5 shows the phosphorylation of EGR-R in agonist-treated cells. Phosphorylation of EGFR by TNF, IL-1, EGF, PDGF (indicated as PGF) and okadaic acid (OA). ³²P-Labeled cultures of fibroblast cells were treated with TNF (100 units/ml), IL-1 (100 units/ml), EGF (50 ng/ml), PDGF (32 ng/ml) or OA (250 nM) for 15 min. Control is indicated by 0. The EGFR was immunoprecipitated with anti-EGFR antibody (Oncogene Science, Manhasset, N.Y., US). The proteins were subjected to 7.5% SDS-PAGE and autoradiography.

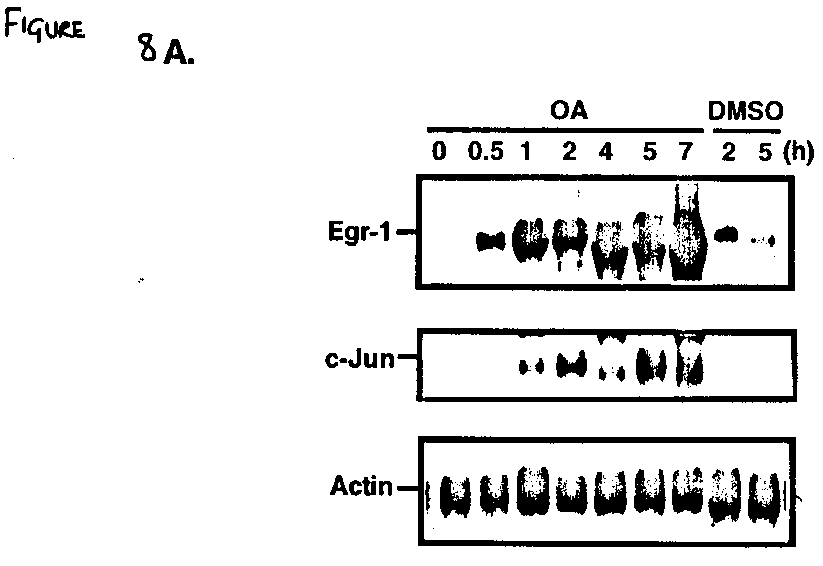

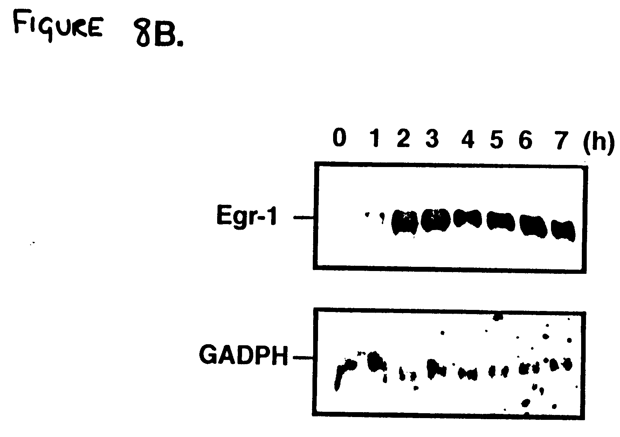

Fig. 6 shows the phosphorylation of cdc2 kinase substrates in fibroblasts after treatment with TNF or okadaic acid. Where c-abl (top panel) or p53 (bottom panel) phosphorylation was measured, cells were treated for the times indicated with 50 units/ml TNF or 100 nM OA. Where RB (middle panel) phosphorylation was measured, cells were treated for 15 min with the indicated doses of TNF (in units/ml) or OA (in nM). Cell lysates were immunoprecipitated with antibodies to either c-abl, RB, or p53 (from Oncogene Science). The immunoprecipitated proteins were subjected to 6 or 10% SDS-PAGE and autoradiography. Note that in the middle panel, the lesser extent of RB phosphorylation observed with 500 nM OA as compared to 100 nM OA is due to the loading of about four times less amount of protein in the 500 nM OA than the 100 nM OA-treated sample to the gel. At either concentration of OA, the ratio of the hyperphosphorylated form of RB is higher than the hypophosphorylated form of RB.

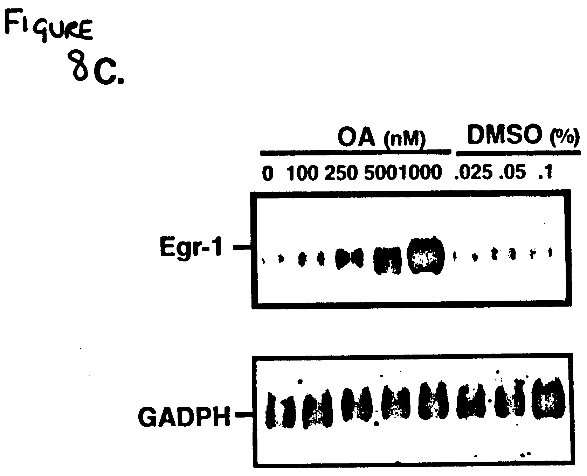

Fig. 7 shows Northern Blot analyses of the effects of TNF and okadaic acid (OA) on Egr-1, c-jun, and IL-6 gene expression. Quiescent human fibroblasts were incubated with TNF (500 units/ml) (a) or OA (500 nM) (b) for 0, 30, 60 min after which total cellular RNA was extracted. Blots of RNA from these cells were hybridized to radiolabeled (10⁶ cpm/ml) Egr-1 cDNA probe. c, the blot in b was stripped and reprobed with c-jun cDNA. Note: Egr-1 mRNA probe was not completely stripped from the blot. The autoradiograph of the blot is presented. d, regularly maintained cells were incubated wither with no additions (control, 0 time) or with OA for 1, 3, 5, 7 h and Northern analysis was carried out with an IL-6 cDNA probe (ATCC, Rockville, MD, US).

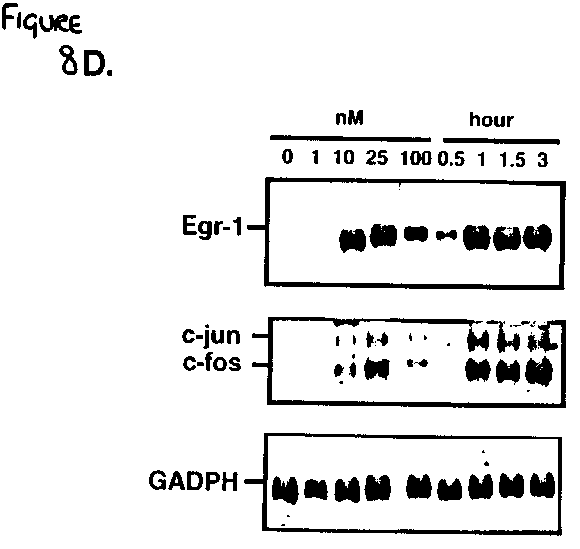

Fig. 8 shows Northern analysis of Egr-1 gene induced by okadaic acid. 10 µg of RNA was applied to each lane and subjected to Northern blot analysis using an Egr-1 specific cDNA probe [1.6 kb Bgl II fragment (Sukhatme et al, Cell 53, 37-48, 1988)]. The same blots were rehybridized with actin or GADPH cDNA for normalizing the amount of RNA loaded. The blots were dried and autoradiographed.
(A). Time course of okadaic acid-induced Egr-1 and c-jun messages in primary human fibroblasts. Quiescent human fibroblasts were induced with 1 µM of okadaic acid for the time periods indicated. The cells were also induced with 0.1% DMSO as a control. The middle panel showed the hybridization of the same blot to plasmid pCMV-jun (Rauscher et al, Genes Dev. 2, 1687-1699, 1988)
(B). Time course of okadaic acid-induced Egr-1 message in Balb/c 3T3 cells. Quiescent Balb/c 3T3 cells were induced with okadaic acid (0.5µM) for the times indicated.
(C). Concentration dependent induction of Egr-1 message by okadaic acid in Balb/c 3T3 cells. Quiescent Balb/c 3T3 cells were induced with the indicated concentrations of okadaic acid for 1 h. As controls, different concentrations of DMSO were included. These corresponded to the amounts present in 0.25 µM okadaic acid (0.025% dimethyl sulphoxide (DMSO)) 0.5 µM okadaic acid (0.05% DMSO) and 1 µM okadaic acid (0.1%).
(D). Concentration dependent and time course induction of Egr-1 message by calyculin A. Quiescent Balb/c 3T3 cells were induced with different concentrations of calyculin A for 1 h. Northern analysis of this dose-dependent induction by calyculin A is shown on the left panel of the figure. Similarly quiescent Balb/c 3T3 cells were also induced with 15 nM of calyculin A for 0.5, 1, 1.5 and 3 h. Northern analysis of this time course induction by calyculin A is shown in the right panel of the figure. The same blot was rehybridized with pCMV-fos (Curran et al, Oncogene 2, 79-84, 1987) and pCMV-jun (Rauscher et al, 1988) shown in the middle panel. The blot was dried and autoradiographed.

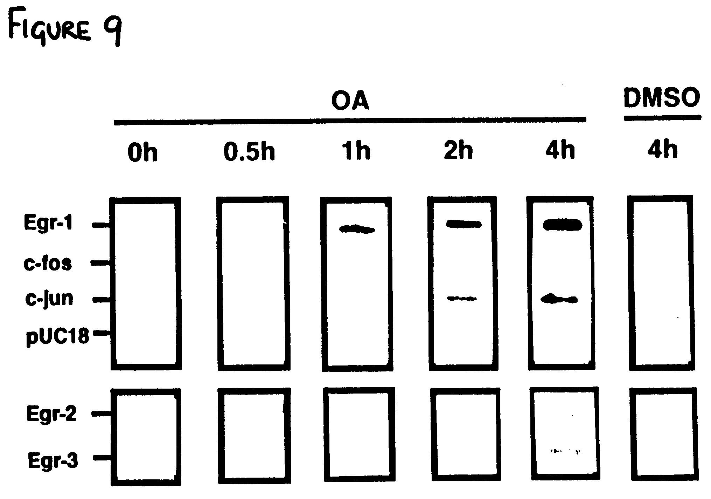

Fig. 9 shows the results of a nuclear run-on assay. Nuclei were isolated from quiescent (0 h) and okadaic acid-treated (0.5 µM) Balb/c 3T3 cells under conditions described in the "Experimental Procedures" of Example 2. Nylon matrix containing the indicated plasmids were hybridized with the in vitro transcribed RNA and then autoradiographed. Control cells were also treated with DMEM containing 0.058 of DMSO and RNA from these cells were also transcribed and hybridized to the various plasmids indicated.

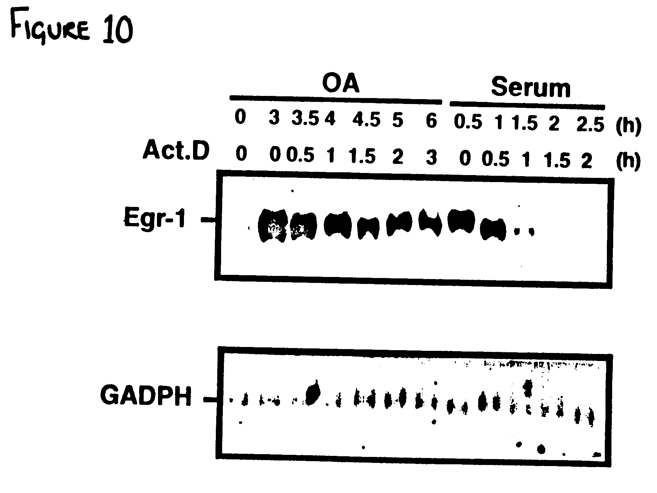

Fig. 10 shows the stabilization of Egr-1 mRNA by okadaic acid. (A). Quiescent Balb/c 3T3 cells were incubated with either 0.5 µM of okadaic acid or 20% serum for 3 h or 0.5 h, respectively. Actimomycin D (final concentration being 25 µg/ml) was then added. The cells were then harvested at 0.5, 1, 1.5, 2 and 3 h after the addition of actinomycin D. RNA was isolated from these cells and analyzed by Northern blot. The blot was probed with Egr-1 or GADPH cDNA.

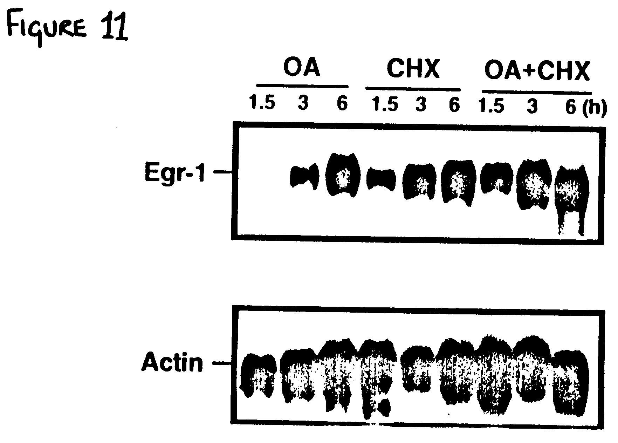

Fig. 11 shows the superinduction of the Egr-1 message in okadaic acid-treated human fibroblasts. Cells were incubated with either 0.5 µM of okadaic acid or 10 µg/ml of cycloheximide as well as with 0.5 µM of okadaic acid and 10 µg/ml of cycloheximide for 1.5, 3 or 6 h. Cells were harvested, RNA isolated and subjected to Northern blot analysis. The blot was probed with Egr-1 or actin cDNA.

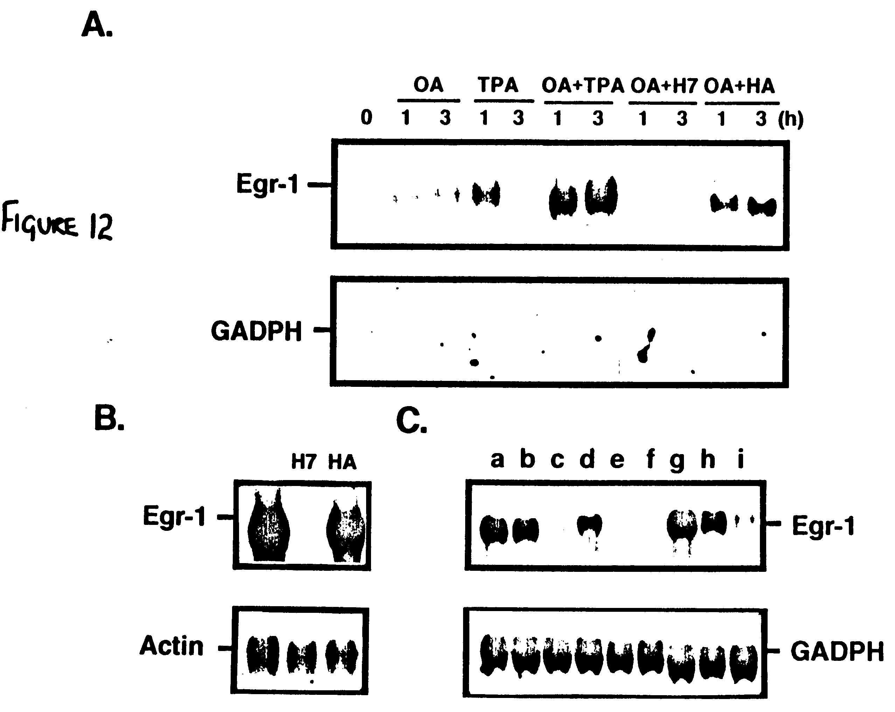

Fig. 12 shows the effect of protein kinase activator and inhibitors on the induction of Egr-1 mRNA by okadaic acid.
(A). Quiescent primary human fibroblasts were incubated with okadaic acid (OA) or TPA for 1 or 3 h. The concentrations of OA and TPA were 0.5 µM and 120 ng/ml, respectively. The concentration of 1-(5-isoquinolinylsulfonyl)-2-methylpiperazine dihydrochloride (H7) and N-(2-guanidinoethyl)-5-isoquinolinesulphonamide dihydrochloride (HA1004) used was 25 µM. The cells were pre-treated for 15 min with H7 or HA1004 before they were induced by OA or TPA for 1 or 3 h. Cells were harvested, RNA was isolated and subjected to Northern blot analysis. The blot was probed with Egr-1 or GADPH cDNA.
(B). As a positive control, primary human fibroblasts were treated with TPA (30 ng/ml) for 1 h (1st lane). The fibroblasts were also pre-treated with H7 (25 µM) or HA1004 (25 µM) for 15 min before treatment with TPA for 1 h (2nd and 3rd lanes respectively). The figure shows Northern blot analysis of RNA isolated from cells subjected to this treatment. The blot was probed with Egr-1 or actin cDNA.
(C). Quiescent Balb/c 3T3 cells were incubated with OA, TPA or dBt-cAMP for 1 h or pre-treated with H7 (25 µM) or HA1004 (25 µM) for 15 min and then incubated with each of the agonists for 1 h. Cells were harvested, RNA was isolated and subjected to Northern blot analysis. The concentration of each agonist used was as follows: lane a: 0.5 µM of OA, lane b: 100 ng/ml of TPA, lane c: 1 mM of dBt-cAMP, lane d: H7 plus OA (0.5 µM), lane e: H7 plus TPA (100 ng/ml), lane f: H7 plus dBt-cAMP (1 mM), lane g: HA1004 plus OA (0.5 µM), lane h: HA1004 plus TPA (100 ng.ml), lane i: HA1004 plus dBt-cAMP (1mM). The blot was probed with Egr-1 or GADPH cDNA.

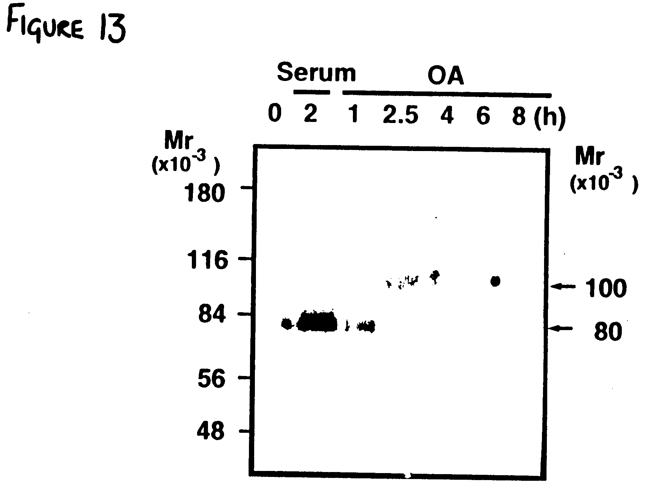

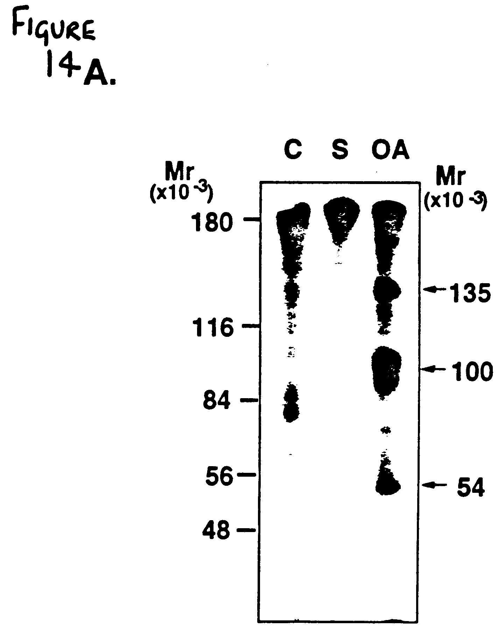

Fig. 13 shows Western blot analysis of Egr-1 protein from primary human fibroblasts induced by okadaic acid. Quiescent primary human fibroblasts were incubated with MEM containing 0.5 µM of okadaic acid for the times indicated. As a positive control, the quiescent cells were also incubated with medium containing 20% fetal calf serum for 2 h. Total cellular proteins were extracted and subjected to Western blot analysis as described previously (Cao et al, Mol. Cell. Biol. 10, 1931-1939, 1989). The blot was reacted with anti-Egr-1 polyclonal antibody (R5232).

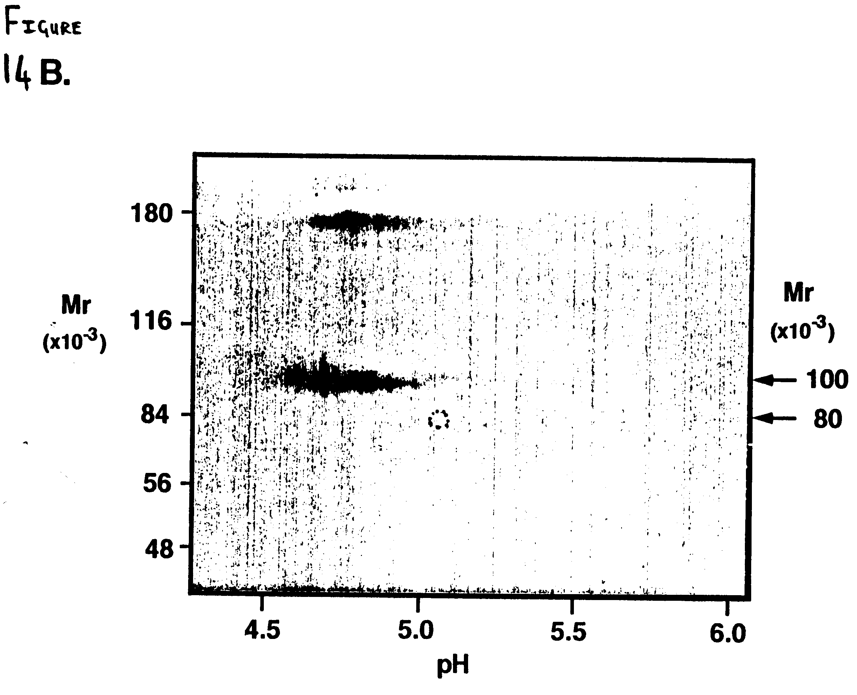

Fig. 14 shows the immunoprecipitation of [³²P]-labeled Egr-1 protein from Balb/c 3T3 cells.
(A). Quiescent Balb/c 3T3 cells were labeled with [³²P]-orthophosphate and incubated with either 20% fetal calf serum or okadaic acid (0.5 µM) for 2 h as described in "Experimental Procedures" of Example 2. Egr-1 protein(s) was immunoprecipitated by anti-Egr-1 antibody (R5232) and subjected to 7.5% SDS-PAGE. The dried SDS-gel was autoradiographed. lanes C, S and OA show immunoprecipitated proteins derived from quiescent cells, serum and okadaic acid-treated cells respectively.
(B). The immunoprecipitated Egr-1 protein(s) from okadaic acid-treated [³²P]-labeled Balb/c 3T3 cells was also subjected to 2-D gel electrophoresis as previously described (Guy et al, J. Biol. Chem. 266, 14343-14352, 1991). The analysis was concurrently repeated with proteins immunoprecipitated from control quiescent [³²P]-Labeled cells. The gels were dried and autoradiographed. The two autoradiographs were superimposed on each other. The dotted circle indicates the position of the weakly phosphorylated Egr-1 protein derived from the control quiescent cells.

### EXAMPLE 1

### EXPERIMENTAL PROCEDURES

Cell Cultures and Reagents - Human diploid fibroblasts, FS-4, 2343 gifts from Dr. J. Vilcek (New York University). They were grown in tissue culture flasks and maintained with minimal essential medium (MEM) supplemented with 10% fetal bovine serum (Hyclone, Logan, UT, US) herein referred to as regular medium. Human WISH cells were purchased from the American Type Culture Collection. Recombinant human TNF and IL-1 with specific activities of 2 x 10⁷ units and 1 x 10⁸ units/mg protein, respectively, and of purities of ≧99% were purchased from Genzyme Corp. (Cambridge, MA, US). Phorbol 12-myristate 13-acetate, bradykinin, forskolin, dibutyryl-cyclic AMP (dBt-cAMP) and FGF were from Sigma, Okadaic acid was purchased from Biomol (Plymouth Meeting, PA, US). Radioactive [³²P]P was from Du Pont-New England Nuclear. Recombinant human a and gamma interferons were gifts from Dr. Paul Trotts of Schering Corp. (Bloomfield NJ, US) and Dr. W. Berthold (Karl Thomae GmbH, Biberach, Germany). The purities of these interferons are ≧ 99%.

Radiolabeling of Cells and Cell Lysis - For labeling with [³²P]orthophosphate cells were plated onto 90 mm plastic tissue culture dishes and incubated for 3 days for the cells to become confluent. Immediately before labeling, the media was removed and the cells were washed twice with 5 ml of phosphate- and serum-free media (150 mM NaCl, 5 mM MgCl₂, 5 mM KCl, 1.6 mM CaCl₂, 0.5% glucose, 10 mM Tris, 0.1% bovine serum albumin, pH 7.4) before the addition of 1 mCi/ml of [³²P]orthophosphate in 3 ml of the same media to each plate. The plates were incubated at 37°C in a humidified CO₂ incubator for 2 h before the addition of the okadaic acid or other agonists at the concentrations and for the times indicated in the figures and table. The agonists were reconstituted in aqueous buffered solution or in dimethyl sulfoxide. Appropriate controls which were run with buffer or dimethyl sulfoxide in medium alone showed no detectable differences in the resulting autoradiographs. At the completion of labeling the media was removed, and the cells were rapidly washed twice with 5 ml of ice-cold phosphate-buffered saline before being solubilised in 200 µl of cytosol extraction buffer (10mM Tris-HCl, 50 mM EDTA, 50 mM NaCl, 30 mM sodium pyrophosphate, 50 mM NaF, 100µM Na₃VO₄, 0.65% Nonidet P-40, 2 mM leupeptin, 2mM phenylmethylsulfonyl fluoride, pH 7.4). The lysate was collected and centrifuged for 5 min at 14,000 rpm. The pellet containing the nuclear fraction (including the cytoskeletal-membrane fractions) was separated from the cytosolic fraction. Both fractions were lyophilized.

Two-dimensional Gel Electrophoresis - The lyophilized fractions (each of approximately 12 µg) were subjected to isoelectric focusing for 18,000 V/h with pH 3-10 ampholytes using the Millipore Investigator two-dimensional electrophoresis system (Millipore). Following this, the first-dimension gel was extruded and equilibrated in SDS buffer for 2 min before being loaded onto the second dimension 12.5% SDS-polyacrylamide gel. The gel was fixed, dried and ³²P-labeled polypeptides were located by autoradiography by using intensifying screens at -80°C. For measurement of the pH profile, carbamylated protein standards (Pharmacia LKB Biotechnology Inc.) were co-run on duplicate gels and related to proteins of known pI on previously calibrated samples. Molecular weight marker proteins from Sigma were detected by Coomassie Blue staining and marked on the autoradiogram. ³²P-Labeled 27 hsp complex prepared from human fibroblasts heat shocked at 43°C for 30 min was also used as a marker.

Quantitative Determination of Protein Phosphorylation - The amount of protein phosphorylation on autoradiographs derived from cytosolic membrane or antibody-precipitated preparations containing equal amounts of protein were analyzed by computerized densitometry using a Visage 2000 Image analysis system (BioImage Products, Ann Arbor, MI, US).

Preparation of RNA and Northern Analysis - Human fibroblasts (4 x 10⁶) were seeded in 175cm² tissue culture flasks and incubated at 37°C for 6 days in regular medium. The cells were fed with regular medium on day 3. On day 6 the medium was replaced by minimal essential medium containing 0.25% fetal calf serum and incubated for another 48 h to maintain the cells in a quiescent state. Quiescent or regularly maintained cells were treated with a specific agonist for the times indicated (see Fig. 7). The cells were harvested and total cytoplasmic RNA isolated by a one-step guanidinium thiocyanate/phenol/chloroform method. RNA was subjected to electrophoresis through 1.3% agarose gels containing 6.5% formaldehyde and then transblotted to Hybond-N membrane (Amersham, Buckinghamshire, UK). The blots were subjected to UV cross-linking and hybridized in a solution containing 0.5 M sodium phosphate buffer, pH 7.2, 7% SDS, 1% bovine serum albumin, and 1 mM EDTA. Hybridization was carried out at 65°C for 18 h with a ³²P-labeled probe (final concentration of 2 x 10⁶ cpm/ml) generated by random priming on DNA templates. A BglII fragment of 1.6 kb corresponding to nucleotides 302-1958 of Egr-1 cDNA was used in the hybridization. The IL-6 cDNA probe was from the American Type Culture Collection (Rockville, MD, US) and the c-jun cDNA probe was from Dr. D. Carter of our institute. The membranes were exposed to x-ray films at -85°C for 14 h to 7 days and rehybridized with β-actin probe. The blots were autoradiographed and quantitated by the Visage 2000 Image system (BioImage Products, Ann Arbor, MI, US).

### RESULTS

### Changes in Early Phosphorylation of Cellular Proteins in Cells Treated with Okadaic Acid, TNF, or Other Agonists -

The ³²P-labeled protein spots are resolved and detected by autoradiography of the high definition two-dimensional gel electrophoresis of cytosolic extracts prepared from ³²P-labeled human fibroblasts. The radioactive spots (each representing one or more phosphoproteins) are analyzed by computerized densitometry. Each spot is arbitrarily assigned a number. In addition, the intensity of each resolved spot derived from extracts of cells previously treated with an agonist is expressed relative to a basal level of phosphorylation in the absence of agonist. These changes in the level of phosphorylation are matched against several internal standards during the computerized densitometry analysis. A typical example of the autoradiography of the two-dimensional gel analysis of the ³²P-labeled phosphoproteins derived from the cytosolic or nuclear/cytoskeletal membrane fractions prepared from cells treated with either TNF (50 units/ml) or okadaic acid (200 mM) for 15 min is shown in Fig. 1. The prominent changes to protein phosphorylation in the ligand activated cells are indicated (arrows). In the cytosolic fraction, okadaic acid induced the same changes (about 95% identity) in early protein phosphorylation as TNF (Fig. 1, b and c). The phosphorylation pattern induced by IL-1 is not shown in Fig. 1. Okadaic acid also induced almost identical changes (as identified by computerized densitometry) in protein phosphorylation of the 46-50 proteins in the nuclear/cytoskeletal membrane fraction as did TNF treatment (Fig. 1, e and f). A summary of the changes in cytosolic protein phosphorylation is listed in Table I, providing a composite pattern of changes of protein phosphorylation in these fractions derived from human fibroblasts activated for 15 min by either TNF, okadaic acid, TPA, or combinations of these ligands. In the numerous experiments performed okadaic acid is shown to mimic TNF in inducing changes in the early phophorylation of about 60 and the dephosphorylation of six proteins in the cytosolic fraction during the first 15 min of ligand treatment (Table 1). Combinations of optimal doses of okadaic acid (200 mM) and TNF (50 units/ml) in the treatment of fibroblasts do not produce an additive or synergistic effect on these changes (Table I). TPA (an activator of protein kinase C) treatment of human fibroblasts produced a pattern of phosphorylation changes quite different from that produced by okadaic acid or TNF (Table 1). TPA in combination with either okadaic acid or TNF produced a pattern of phosphorylation changes which represents the sum of complementary changes produced by each agonist (Table 1). These results indicate that okadaic acid and TNF produce early changes in protein phosphorylation through a common pathway which is different from that of TPA-induced activation of protein kinase C. Similarly, the pattern of protein phosphorylation induced in cells treated by bradykinin, cAMP agonists, EGF, or IFN-α is different from that induced by okadaic acid or by TNF/IL-1 (data not shown). Furthermore, treatment of human fibroblasts by IFN-gamma did not produce measurable changes in early protein phosphorylation.

A number of the approximately 66 proteins in the cytosol fraction and the 46-50 proteins in the nuclear/cytoskeletal membrane fraction which undergo the early phosphorylation changes have now been identified by either using marker proteins or by their molecular weights and isoelectric points. They include the 27 hsp complex (identified as 165, 172 and 176 in Table I), stathmin (as S[27] in Fig. 1, b and c), nucleolin (as N[28] in Fig. 1, 3 and f), and myosin light chain (MLC in Fig. 1, e and f). The phosphorylation of these proteins is concordantly enhanced in cells activated by TNF (Fig. 1, b and e), by okadaic acid (Fig. 1, c and f) or by IL-1 (not shown).

In a separate experiment using m⁷GTP-Sepharose to isolate the eIF-4E from okadaic acid-treated human fibroblasts by procedures previously reported (Guy et al, 1991) for TNF and IL-1, okadaic acid also enhanced the phosphorylation of this protein after 15 min by 2-3 fold (Fig. 2). Okadaic acid treatment of cells was extended to WISH cells and again produced almost identical changes in early protein phosphorylation as TNF (not shown), indicating that the mimicry of the early events of TNF signal transduction by okadaic acid is not confined to primary human fibroblasts.

It is important to note that though the TNF or IL-1 induced changes in protein phosphorylation shown in Fig. 1 and Table I were measured 15 min after ligand treatment, these changes were detectable within 3 min and maximal at about 15 min before returning to basal levels at 45-60 min. By then most of the changes in phosphorylation induced by TNF or IL-1 are not longer measurable.

Dose-response and Time Course of Okadaic Acid-induced Changes in the Phosphorylation of the 27 hsp Complex - The 27 hsp complex is most sensitive to phosphorylation changes induced by okadaic acid and TNF (Fig. 1). The concentration-dependent effect of okadaic acid on changes in phosphorylation of the 27 hsp complex was measured and compared with those in cells treated with 50 units/ml of TNF (Fig. 3). It is apparent from this comparison that treatment of cells with 50 units/ml of TNF for 15 min produces about the same increase in the phosphorylation of the 27 hsp complex as treatment of cells with between 160-320 nM okadaic acid for 15 min (Fig. 3, c-e).

A measurement of protein phosphorylation of the 27 hsp complex was performed in cells treated with 200 nM okadaic acid for 0, 5, 15 and 30 min. This measurement shows proportional increases in phosphorylation of the 27 hsp complex with increasing time of okadaic acid treatment (Fig. 4, a-d). After 30 min of okadaic acid treatment the 27 hsp complex was hyperphosphorylated.

IL-1, TNF, or Okadaic Acid Treatment Stimulate Phosphorylation of the EGF Receptor - One feature of TNF and IL-1 action is the transmodulation of the EGFR via phosphorylation. Okadaic acid was added to human fibroblasts to see if it can also mimic this effect of TNF and IL-1. ³²P-Labeled human fibroblasts were treated with ligand (TNF, IL-1, EGF, PDGF, or okadaic acid) for 15 min and extracted for analysis. The extracts were immunoprecipitated with anti-EGFR antibodies. The immunoprecipitated EGFR derived from variously treated fibroblasts was analyzed by SDS-PAGE and autoradiography. Okadaic acid significantly stimulates the phosphorylation of the EGFR to an even greater extent than does TNF, IL-1, EGF or PDGF (Fig. 5).

Phosphorylation of cdc2 Kinase Substrates in Cells Treated with IL-1, TNF, or Okadaic Acid - It is noted from Fig. 1 (e and f) that the phosphorylation of myosin light chain and nucleolin are enhanced by TNF and okadaic acid treatment. Both proteins are known to be substrates of cdc2 kinase. In this connection, a question was raised as to whether other cdc2 kinase substrates which are known to be phosphorylated by okadaic acid treatment are also effected in TNF-treated human fibroblasts. Three substrates to cdc2 kinase, namely c-abl, RB, and p53, were tested. Like okadaic acid, TNF induced the phosphorylation of all three cdc2 kinase substrates in human fibroblasts. Okadaic acid or TNF enhanced the phosphorylation of c-abl, RB and p53 (Fig. 6). The results also show that the RB proteins are found in two forms, one having slightly higher molecular mass (115 kDa) than the other (110 kDa). The 115-kDa protein is likely the hyperphosphorylated RB protein, whereas the 110-kDa protein represents the hypophosphorylated form. Like okadaic acid treatment of cells, TNF increases the ratio of the hyperphosphorylated RB protein to the hypophosphorylated form of RB (Fig. 6). Similarly the p53 protein is present in two very closely associated forms, the slightly higher molecular mass species being more heavily phosphorylated than the slightly lower molecular mass species. Like TNF-treated cells, okadaic acid-treated fibroblasts showed 3- to ≧ 5-fold higher amounts of the hyperphosphorylated RB and p53 protein than the lesser phosphorylated forms of these proteins.

Induction of Egr-1, c-jun. and IL-6 Genes by TNF and Okadaic Acid - Having shown the early changes in protein phosphorylation in the cytosol and nuclear/cytoskeletal membrane fractions of okadaic acid-, TNF-, and IL-1 treated fibroblasts, we investigated the effects of okadaic acid on gene expression. TNF is known to induce eight genes in primary human fibroblasts. Recently we observed the induction of an immediate early response gene, the Egr-1 gene, in primary human fibroblasts in response to TNF. The induction of expression of three TNF/IL-1 inducible genes by okadaic genes selected were Egr-1, c-jun and IL-6. Like TNF, okadaic acid effectively induced all these genes, Egr-1 was rapidly induced within 30 min and c-jun within 60 min. of okadaic acid treatment, whereas IL-6 was induced much later at 7h. after okadaic acid treatment (Fig. 7).

### EXAMPLE 2

### EXPERIMENTAL PROCEDURES

Cell Cultures and Reagents. Human diploid foreskin fibroblasts were a gift from Dr J Vilcek (New York University, New York). The cells were grown in MEM supplemented with 10% fetal bovine serum (Hyclone, Logan, UT, US), Mouse Balb/c 3T3 cells were purchased from the American Type Culture Collection and grown in Dulbecco's MEM (DMEM) supplemented with 10% fetal bovine serum. Okadaic acid, calyculin A, H7, and HA1004 were purchased from Biomol Research Laboratories (Plymouth Meeting, PA, US). TPA, puromycin and dBt-cAMP were purchased from Sigma (St Louis, MO, US) 1 mM stock solutions of okadaic acid or calyculin A were solubilized in DMSO. [³²P]orthophosphate and other radioactive chemicals were purchased from Du Pont-New England Nuclear (Boston, MA).
Preparation of RNA and Northern Analysis. Balb/c 3T3 cells were grown to near confluency in DMEM supplemented with 10% fetal calf serum. The cells were then maintained in DMEM without serum for 48 h followed by treatment with the indicated agonists for the times indicated. Similarly human fibroblasts were maintained in MEM supplemented with 10% fetal bovine serum. Nearly confluent human fibroblasts were maintained for 2 days in MEM supplemented with 0.25% fetal bovine serum before treatment with agonists. The cells were harvested and total cytoplasmic RNA was isolated and subjected to Northern analysis as previously described (Chomczynski et al, Anal. Biochem. 162, 156-159, 1987). The blots were autoradiographed and quantitated with the aid of the Visage 2000 Image System (BioImage Products, Ann Arbor, MI, US).
Nuclear Run-on Analysis of Transcription. Nuclei were isolated from quiescent or okadaic acid-treated Balb/c 3T3 cells following the procedures previously reported (Marzluff, Methods Cell Biol. 19, 317-331, 1978) and recently modified (Murphy et al, Mol. Endocrinol. 4, 1051-1059, 1990). Accordingly, monolayers of fibroblasts grown in 175 cm² plastic tissue culture flasks were washed twice with about 100 ml of phosphate-buffered saline (PBS). The washed cells were lysed in 4 ml of cold NP-40 lysis buffer pH 7.0 (containing 10 mM HEPES, 10 mM NaCl, 3 mM MgCl₂, 0.5% NP40 and 50 units/ml human placental ribonuclease inhibitor) on ice for 5 min and then centrifuged 500 x g for 5 min. The nuclear pellets were resuspended in 100 µl glycerol storage buffer pH 7.9 (containing 50 mM HEPES, 5 mM MgCl₂, 40% glycerol and 500 units/ml human placental ribonuclease inhibitor) and frozen at -80°C.

For the in vitro transcription reaction, 100 µl of nuclei were thawed on ice, and incubated immediately with 20 µl of 10 x reaction buffer (1.5 M NaCl, 25 mM MgCl₂, 50 mM magnesium acetate, 10 mM MnCl₂, 20 mM DTT, 1.25 mM EDTA, 5 mM ATP, 5 mM GTP, 5 mM CTP, 20 mM creatine phosphate, 30 units/ml creatine phosphokinase and 5 mg/ml heparin) and with nucleotide 5'-diphosphate kinase (final concentration 12 units/ml), RNAse inhibitor (final concentration 500 units/ml) and 250 µCi of [³²P]UTP. Water was then added to bring the final volume to 200 µl.

After incubation at 30° for 1 h, vanadylribonucleoside complex (final concentration of 2 mM) was added along with 600 units of DNAse I and incubated at 30°C for another 30 min. The digest was incubated with 2 µg of proteinase K in solution containing 0.15 M Tris-HCl, pH 7.4, 37.5 mM EDTA, and 1.5% SDS at 42° for 30 min. RNA was isolated from the incubated preparations by extraction with phenol-chloroform-isoamyl alcohol (24:24:1) and the extract precipitated with cold ethanol. The precipitated RNA was resuspended in 45µl of ice-cold 0.2 M NaOH, incubated on ice for 10 min, and neutralized with 5µl of 2.4 HEPES. RNA solution was passed through Nick^{TM} column (Pharmacia LKB Biotechnology, Uppsala, Sweden) previously equilibrated with RNase-free water).

The radioactivity of the in vitro transcribed RNA was determined and equal amounts of radioactive RNA from uninduced and okadaic acid treated cells were used to hybridize to specific plasmid (5 µg) previously applied to a nylon matrix with the aid of a slot blot applicator (Schleicher & Schuell, Keene, NH, US). The nylon matrix containing the plasmid was pretreated in 0.5 M sodium phosphate (pH 6.8), 7% SDS, and 1 mM EDTA at 65°C for 6 h before hybridization with the [³²P]-labeled RNA for 48 h. The hybridized filters were exposed to x-ray film for 1-3 days.
Radiolabeling cells and immunoprecipitation. Balb/c 3T3 mouse fibroblasts were seeded in 35 mm tissue culture dishes and grown to near confluency. They were then maintained in DMEM without fetal calf serum for 48 h to lead them into quiescency. The medium was then removed and the cells were washed twice with 5 ml of phosphate- and serum-free medium (150 nM NaCl, 5 mM MgCl₂, 5 mM KCl, 1.6 mM CaCl₂, 0.5% glucose, 10 mM Tris-HCl, 0.1% bovine serum albumin, pH 7.4). The cells were incubated in 2 ml of the same medium with 0.5 mCi/ml of [³²P]-orthophosphate for 2 h.

Okadaic acid or calyculin A was then added to a final concentration of 0.5 µM or 25 nM respectively and incubated for another 2 h. The medium was removed and the cells were rapidly washed twice with 5 ml of ice-cold PBS before being solubilized in 200 µl of extraction buffer (10 mM Tris-HCl, 50 mM EDTA, 50 mM NaCl, 30 mM sodium pyrophosphate, 50 mM NaF, 100 µM Na₃VO₄, 0.65% Nonidet P-40, 2 mM leupeptin, 2 mM phenyl-methylsulfonyl fluoride, pH 7.4). Cell lysates were immunoprecipitated with polyclonal antibody against Egr-1 (R-5232) according to the procedure described (Cao et al, Mol. Cell. Biol. 10,1931-1939, 1989). The immunoprecipitated proteins were analyzed by 7.5% SDS-PAGE as well as by two-dimensional gel electrophoresis as described previously (Guy et al, J. Biol. Chem. 266, 14343-14352, 1991).

### RESULTS

### Kinetics and Dose Dependency of Mouse and Human Egr-1 mRNA Induction by Okadaic Acid and by Calyculin A

Quiescent primary human fibroblasts or mouse Balb/c 3T3 were incubated with MEM or DMEM containing 1 µM or 0.5 µM of okadaic acid respectively for the times indicated in Fig. 8A and B. RNA isolated from these cells were subjected to Northern blot analyses. The results in Fig. 8A and B show that okadaic acid rapidly induces the Egr-1 mRNA in both human and mouse fibroblasts within 1 h. The induction of this mRNA is maximally increased by about 2 h and the concentration of Egr-1 mRNA is maintained at these levels (30 to 40 fold over control) for up to 7 h. The induction is dependent on the concentration of okadaic acid (Fig. 8C). The concentration of DMSO used in these experiments was 0.05% since okadaic acid was initially dissolved in DMSO. Control experiments with MEM containing 0.05% DMSO induce a small amount of Egr-1 mRNA not comparable to the amount induced by okadaic acid. Furthermore, the induction of Egr-1 mRNA by DMSO is not dose-dependent (Fig. 8C).

The induction of Egr-1 mRNA by another potent protein phosphatase inhibitor calyculin A, which is structurally unrelated to okadaic acid, was also tested. Calyculin A strongly induced the expression of Egr-1 mRNA in mouse Balb/c 3T3 fibroblasts with kinetics similar to that observed with okadaic acid and the induction increased dose-dependently with the concentration of calyculin A up to 25 nM (Fig. 8D). Notably, calyculin A is a stronger inducer of the Egr-1 gene because 10 nM of calyculin A can induce an equivalent fold increase in Egr-1 mRNA as 1000 nM of okadaic acid (Fig. 8C and D). Immediate-early genes c-fos and c-jun were also induced by calyculin A as shown in Fig. 8D. These genes were also recently reported to be induced by okadaic acid (Schontahl et al, Oncogene 6, 423-430, 1991; Thevenin et al, J. Biol. Chem. 266, 9363-9366, 1991).

### Okadaic Acid Activates the Transcription of Egr-1 mRNA

Quiescent Balb/c 3T3 fibroblasts were incubated with DMEM containing 0.5 µM okadaic acid for the times indicated in Fig. 9. The cells were washed, harvested and nuclear fractions prepared for the nuclear run-on analyses. The results of these analyses (Fig. 9) show the kinetics of transcription of the Egr-1 mRNA in the isolated nuclei. A trace of newly transcribed Egr-1 mRNA was detectable as early as 0.5 h after the cells were exposed to okadaic acid. By 1 h significant amounts of Egr-1 mRNA were transcribed, reaching a maximum by 4 h. The concentration of newly transcribed Egr-1 mRNA was decreased 6 h after okadaic acid treatment (data not shown). Other immediate early genes like c-jun and related genes Egr-2 and Egr-3 were also transcribed with similar kinetics but the amount of transcripts was not as high as the Egr-1 gene. The c-fos gene was also transcribed but at a comparatively low level. Controls performed with pUC18 plasmid did not hybridize to the radiolabeled mRNA. The results indicate that okadaic acid activates the transcription of Egr-1 mRNA in mouse Balb/c 3T3 fibroblasts.

### Stability of Okadaic Acid-induced vs Serum-induced Egr-1 mRNA

Quiescent mouse Balb/c 3T3 fibroblasts were incubated with DMEM containing either 0.5µM okadaic acid or 10% fetal calf serum for the times indicated in Fig. 10. A stock solution of actinomycin D was added at the times indicated in Fig. 10 to the okadaic acid or serum stimulated cells such that the final concentration of actinomycin D was 25 µg/ml [a concentration known to strongly inhibit RNA synthesis in these fibroblasts (Schonthal et al, 1991)]. Cells were harvested at the indicated time points and extracted for RNA and the RNA subjected to Northern blot analysis (Fig. 10). The results indicated that when cells were treated with actinomycin D to block the synthesis of the new Egr-1 transcripts, the serum-induced Egr-1 mRNA rapidly disappears from the cells whereas the okadaic acid-induced Egr-1 mRNA remains stable and is detectable in the cells up to 5.5 h after actinomycin D treatment. Estimation of the turn-over of the Egr-1 mRNA indicated a half life of about 12 min for the serum-induced and about 120 min for the okadaic acid-induced Egr-1 message (calculated from the data in Fig. 10).

### Effect of Protein Synthesis and Protein Kinase Inhibitors on Okadaic Acid-indicated Egr-1 Gene

Quiescent human fibroblasts were stimulated with MEM containing 0.5 µM okadaic acid in the presence or absence of cycloheximide (10 µg/ml) for the times indicated in Fig. 11. Notably cycloheximide alone induced Egr-1 mRNA. Treatment of cells with cycloheximide enhanced the induction of Egr-1 mRNA by okadaic acid at 1.5 and 3 h (Fig. 11). Similarly puromycin, like cycloheximide, also "superinduces" the induction of the Egr-1 mRNA by okadaic acid (data not shown). These results suggest that the induction of Egr-1 message by okadaic acid can occur even though cellular protein synthesis is inhibited. However, in view that protein phosphatase 1 (PP1) and protein phosphatase 2A (PP2A) regulate enzymes associated with cellular protein synthesis (Cohen, Annu. Rev. Biochem. 58, 453-508, 1989) the effect of okadaic acid on protein synthesis in mouse and human fibroblasts was also measured. We observed that 0.5 µM okadaic acid treatment of cells had little measurable effect on total cellular protein synthesis during the first 2 h. However, it began to partially inhibit protein synthesis in these cells by up to 50% at 4 h (data not shown). By then the Egr-1 mRNA message was already induced. A corollary to this is a comparison of the amount of ³⁵S-methionine labelled Egr-1 protein immunoprecipitated by R5232 antibodies from protein phosphatase inhibitor-treated and control cells. Significantly, a higher amount of ³⁵S-methionine labeled Egr-1 protein (4 to 5 fold higher) in immunoprecipitated from the 2 h okadaic acid or calyculin A-treated cells than from the control cells which contain a small but detectable amount of the Egr-1 protein (data not shown). This suggests the de novo synthesis of Egr-1 protein in response to okadaic acid or calyculin A treatment. Thus, the induction of the Egr-1 gene by phosphatase inhibitors is unlikely to be mediated by their late inhibitory effects on cellular protein synthesis.

Quiescent human or mouse fibroblasts were also stimulated with okadaic acid or TPA in the presence or absence of an inhibitor of protein kinase C (PKC) and protein kinase A (PKA) (H7) or a preferential inhibitor of PKA (HA1004)., The results of this experiment (Fig. 12) indicated that okadaic acid and TPA synergistically induced the expression of the Egr-1 mRNA in human and mouse fibroblasts and that H7, but not HA1004, effectively blocked the induction of Egr-1 message by either induced (Fig 12A, B and C), suggesting a role for both protein kinases(s) and phosphatase(s) in the induction of Egr-1 gene.

### Western Blot Analysis of Proteins from Okadaic Acid-treated Primary Human Fibroblasts.

Cellular proteins were extracted from quiescient, okadaic acid-treated and serum-treated human fibroblasts and subjected to Western blot analysis using rabbit polyclonal Egr-1 antibodies. In the control quiescent cells, traces of Egr-1 protein with molecular masses of 80 kDa and 100 kDa were detected. An approximately 6-fold increase of the 80 kDa protein was measured in the cells 2 h after serum stimulation. An approximately 2-fold increase in the 80 kDa Egr-1 protein was detected in the cells after 1 h okadaic acid treatment. However, at 2.5 h after okadaic acid treatment the Egr-1 protein was mostly detected as the 100 kDa moiety rather than as the 80 kDa protein (Fig. 13). Furthermore, the cellular level of the 100 kDa Egr-1 protein was about 3 to 4 fold higher than the 80 kDa Egr-1 protein found in the control cells suggesting de novo synthesis of Egr-1 in response to okadaic acid treatment. By 6 h after okadaic acid treatment the cellular level of the 100 kDa Egr-1 protein was reduced (Fig. 13). It is possible that the 80 kDa form is the unphosphorylated or a hypophosphorylated Egr-1 protein (Cao et al, 1989) and the 100 kDa form represents the hyperphosphorylated Egr-1. Analysis of the immunoprecipitated [³²P]-labeled proteins from extracts of Balb/c 3T3 cells (described in the next section) is consistent with this explanation.

### Immunoprecipitation of [³²P]-labeled Egr-1 Extracted from Okadaic Acid-treated Balb/c 3T3 Cells

Quiescent mouse Balb/c 3T3 fibroblasts were incubated with [³²P]-orthophosphate for 2 h, then 0.5 µM okadaic acid or 20% fetal calf serum was added and the cells were incubated for another 2 h. The cells were harvested, extracted and immunoprecipitated with Egr-1 rabbit polyclonal antibodies. The immunoprecipitated ³²P-labeled proteins were analyzed by SDS-PAGE as well as by 2-D gel electrophoresis. These gels were dried and autoradiographed (Fig. 14A and D).

In Fig. 14A the autoradiograph shows that at least 3 major phosphorylated proteins (with molecular masses of 54, about 100 and 135 kDa) were immunoprecipitated from okadaic acid-treated cells. The about 100 kDa species detected in okadaic acid-treated cells (lane OA, Fig. 14A) is likely the Egr-1 phosphoprotein whereas the about 135 kDa species may be an antigenically related protein or a protein associated with Egr-1, and the about 54 kDa species is likely degraded Egr-1 phosphoprotein. SDS-PAGE analysis of aged immunoprecipitated samples shows an increase in the amount of the about 54 kDa phosphoprotein with a concomitant decrease in the about 100 kDa phosphoprotein (not shown), an observation consistent with the explanation that the about 54 kDa species is a degradation product.

A low level of Egr-1 phosphoprotein of molecular mass of 80 to 90 kDa was detected in immunoprecipitates from quiescent cells in comparison to a high cellular concentration of the about 100 kDa Egr-1 phosphoprotein in the okadaic acid-treated cells (compare lane C and lane OA of Fig. 14A). No Egr-1 phosphoprotein was found (lane S of Fig. 14A) in the 2 h serum-treated cells. In this connection, we and others have detected Egr-1 phosphoprotein (80 to 90 kDa) 1 h after Balb/c 3T3 cells were treated with serum which disappeared by 2 to 3 h (Cao et al, 1989; Lemaire et al, Mol. Cell Biol. 10, 3456-3469, 1990). This suggests that Egr-1 protein is rapidly phosphorylated and mostly dephosphorylated after 2 h serum stimulation and that the about 100 kDa Egr-1 phosphoprotein observed in the okadaic acid-treated cells is probably a hyperphosphorylated form of Egr-1. Similar results were observed with 25 nM calyculin A-treatment of Balb/c 3T3 mouse fibroblasts (data not shown).

The immunoprecipitated ³²P-labeled phosphoproteins from control and okadaic acid-treated Balb/c 3T3 cells were also analyzed by 2-D gel electrophoresis. The autoradiograph of the 2-D gel analysis of the okadaic acid cell sample was superimposed on the autoradiograph of the 2-D gel analysis of the quiescent cell sample and the composite is shown in Fig. 14B. The Egr-1 immunoprecipitated phosphoproteins from the okadaic acid-treated cells is detected as a phosphoprotein complex with a molecular mass of about 100 kDa and with isoelectric points ranging from pH 4.6 to 5.1 suggesting that the Egr-1 protein is likely phosphorylated at multiple sites. On the other hand, the Egr-1 phosphoprotein from quiescent control cells with a molecular mass of 85 kDa and a pI fo 5.1 (see dotted circle to highlight the faint but detectable phosphoprotein derived from quiescent cells in Fig. 14b) was barely detectable.

## Claims

1. Use of a protein phosphatase inhibitor in the preparation of a medicament for use as a mimic of tumour necrosis factor or interleukin-1.

2. Use according to claim 1, wherein the medicament is for use in the treatment of a tumour selected from an advanced cancer, melanoma, renal cell carcinoma, leukaemia, broncogenic carcinoma, nasopharyngeal carcinoma and Kaposi sarcoma.

3. Use according to claim 2, wherein the medicament is for use after debulking of the main tumour mass.

4. Use of a protein phosphatase inhibitor in the preparation of a medicament for use as a cell differentiation factor.

5. Use according to claim 4, wherein the medicament is for use in the treatment of a leukaemia.

6. Use according to any one of the preceding claims, wherein the protein phosphatase inhibitor is an inhibitor of protein phosphatase 1 or 2A.

7. Use according to claim 6, wherein the inhibitor is okadaic acid or a pharmaceutically acceptable salt, amide or ester thereof.

8. Use according to claim 6, wherein the inhibitor is calyculin A or a derivative thereof.

9. Products containing a protein phosphatase inhibitor and a protein kinase activator as a combined preparation for simultaneous, separate or sequential use in inducing cell differentiation.

10. Products according to claim 9, for use in the treatment of a tumour selected from an advanced cancer, melanoma, renal cell carcinoma, leukaemia, broncogenic carcinoma, nasopharyngeal carcinoma and Kaposi sarcoma.

11. Products according to claim 9 or 10, wherein the protein phosphatase inhibitor is an inhibitor of protein phosphatase 1 or 2A.

12. Products according to claim 11, wherein the inhibitor is okadaic acid or a pharmaceutically acceptable salt, amide or ester thereof.

13. Products according to claim 11, wherein the inhibitor is calyculin A or a derivative thereof.

14. Products according to any one of claims 9 to 13, wherein the protein kinase activator is an activator of protein kinase C.

15. A method of inducing arthritis in an animal, which method comprises administering thereto an amount of a protein phosphatase inhibitor sufficient to induce arthritis therein.
